(19) Europäisches Patentamt
European Patent Office
Office européen des brevets

(11) **EP 1 696 366 B1**

(12) **EUROPEAN PATENT SPECIFICATION**

(45) Date of publication and mention
of the grant of the patent:
**23.12.2009 Bulletin 2009/52**

(51) Int Cl.:
***G06K 9/32*** *(2006.01)* ***G06T 7/00*** *(2006.01)*

(21) Application number: **05101390.2**

(22) Date of filing: **24.02.2005**

(54) **Process of checking the presence of a grid pattern in an X-ray image**

Verfahren zum Prüfen des Vorhandenseins eines Gittermusters in einem Röntgenbild

Procédé de vérifier la présence d'un modèle de grille dans une image de rayon X

(84) Designated Contracting States:
**DE FR GB**

(43) Date of publication of application:
**30.08.2006 Bulletin 2006/35**

(73) Proprietor: **Agfa HealthCare NV**
**2640 Mortsel (BE)**

(72) Inventor: **Behiels, Gert**
**c/o AGFA-GEVEART**
**2640 Mortsel (BE)**

(74) Representative: **Theunis, Patrick et al**
**Agfa-Gevaert N.V.**
**HE / Intellectual Property 3802**
**Septestraat 27**
**2640 Mortsel (BE)**

(56) References cited:
**EP-A- 0 723 762**    **EP-A- 0 962 888**
**US-A1- 2003 152 259**    **US-B1- 6 269 176**

**Description**

FIELD OF THE INVENTION

The present invention relates to a method for detecting grids in a digital image and for obtaining the orientation and frequency of the grid.

BACKGROUND OF THE INVENTION

A commonly used technique to reduce the amount of scattered X-rays in computed radiography, digital radiography as well as classical film-based X-ray systems is the use of anti-scatter grids. These grids are lead foil strips, placed apart at a certain distance in a suitable covering.
There exist different types of anti-scatter grids. In parallel grids, the lead foil strips are parallel, while in honeycomb grids the strips are placed in a honeycomb pattern. The grids are stationary or moving. The use of these grids effectively reduces the radiation scattering but occasionally introduces artifacts such as grid lines into the image.
In a moving system, the motion of the grids removes the grid lines in the image. However, in some circumstances e.g. short exposure time or malfunctioning of the system, the artifacts remain in the image. With stationary grids, the grid lines are almost always visible.
If the image is formed digitally or converted afterwards to a digital image representation, Moiré artefacts may appear when displaying the image at a certain scale. These low frequent Moiré artefacts are mostly disturbing and should be eliminated. Before displaying the image, the grid lines, if present in the image, should be removed. Because the removal algorithms of such grid lines is a time consuming operation, a need exists for a robust method to detect grids in the image so that the processing algorithms for removing grids are only applied when a grid has been used.
US-B1-6 269 176 discloses a method of detecting an anti-scatter grid in digital radiography. A number of classification features are detected such as peak location, peak magnitude, total energy, grid orientation. The extracted features are classified in a classification algorithm.
EP-A-0 723 762 also discloses a method and system for detecting grids in a digital image. The method comprises a high pass filtering operation on a sample of pixels for filtering out the image from the sampled pixels; and performing a statistical F-test operation on the results of the high pass filter operation for determining when the digital image contains grids.

SUMMARY OF THE INVENTION

The above-mentioned aspects are realised by a method as set out in claim 1.
Specific features for preferred embodiments of the invention are set out in the dependent claims.
The present invention provides a method for detecting grids and retrieving information about these grids. This information can be presented to a grid suppression algorithm.
The method is made robust through the use of several classification features which are extracted from the captured image.
These classification features are presented to a classification algorithm which determines whether or not a grid is present. If a grid is present, information about the grid is presented. This information comprises at least one of the orientation of the grid and the frequencies of the grid patterns which are visible in the image.
An example of a classification feature is the percentage of image regions, perpendicular to the grid direction, which score better for a given measure than regions parallel with the grid.
A suitable measure is the maximum power spectrum magnitude at high frequencies. Regions which have a better measure than an average of the measure in the parallel direction to the grid, are taken regions where the grid is most likely best visible. This average of the parallel direction is just an estimate of the measure if no grid was present in the image.
Another example of a classification feature is the percentage of how many of these image regions have their maximum power spectrum magnitudes located around the same frequency.
A third example of a classification feature is the ratio of the averaged power spectrum magnitude at the most likely frequency to the averaged power spectrum magnitude of the neighbouring frequencies.
A fourth example of a classification feature is the corresponding period of the most likely grid frequency in the image.
The classification algorithm is preferably implemented with support vector machines. The input for training of the algorithm consists of an array of the above-mentioned features and the information whether a grid is present.
Support vector machines are for example described in "An introduction to support vector machines and other kernel-based learning methods, N. Cristianini and J. Shawe-Taylor, Cambridge University Press, 2000, ISBN 0 521 78019 5".

After training, the classification rules are stored.

When a new image is presented to the method for detection, first the above-mentioned features are extracted from the new image, the classification rules are loaded and the decision of the grid's presence is made by the classification algorithm.

The proposed method easily allows the extension of the feature list to make the method more robust if the failure rate with these features is not acceptable.

Further advantages and embodiments of the present invention will become apparent from the following description and drawings.

BRIEF DESCRIPTION OF THE DRAWINGS

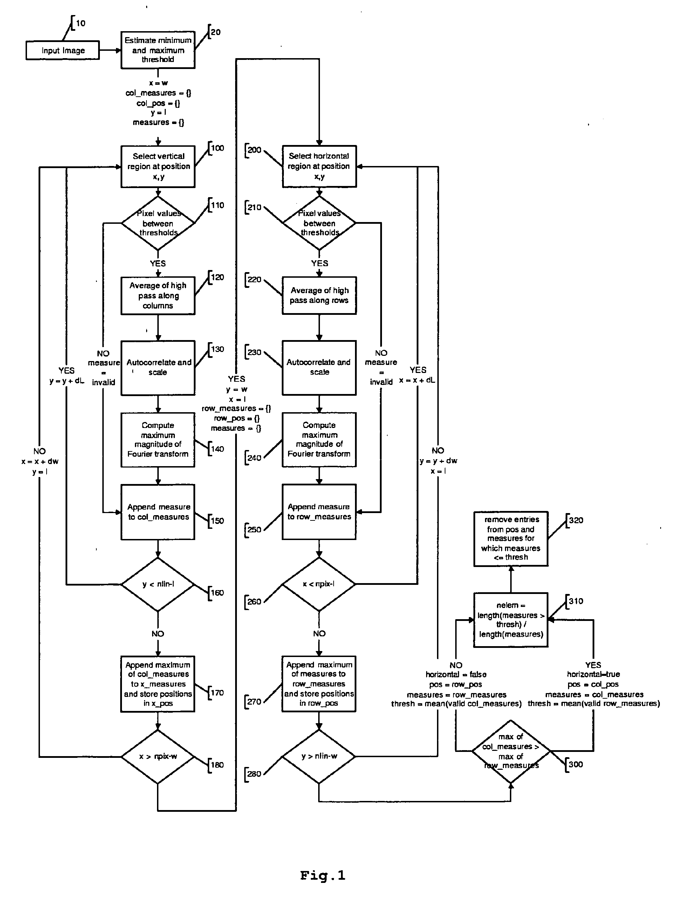

Figure 1 illustrates an embodiment of an algorithm according to the present invention which determines in which parts of the image the grid is most likely visible and which extracts a first feature and decides in which direction the grid is most likely positioned.

Figure 2 illustrates an embodiment of an algorithm according to the present invention for the computation of other classification features from the image parts which are determined in the previous step.

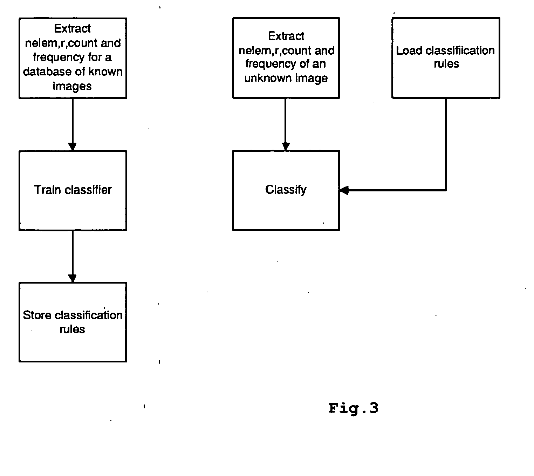

Figure 3 shows a flowchart regarding the training and use of the classification algorithm.

Figure 4 shows an image with a horizontal grid. The locations where the grid is most visible are indicated with circles. The image locations indicated with diamonds correspond to positions where the maximum magnitude of the Fourier spectrum does not exceed and average maximum magnitude of the Fourier spectrum of image regions in the horizontal direction.

Figure 5 shows a histogram of the relative frequencies at the locations with the circles in figure 4 and the average magnitude of the Fourier spectrum at these locations.

Figure 6 shows the ratio of the average magnitude of the Fourier spectrum to a median filtered version of this average magnitude.

DETAILED DESCRIPTION OF THE INVENTION

The input of the method is a digitized radiation image, e.g. a medical image such as an X-ray image. This image is for example obtained by capturing X-rays, generated by an X-ray tube and attenuated by the patient or object to be examined, on a radiation detector such as film or a photostimulable phosphor screen, a CMOS detector or the like. Although the present invention will be explained with respect to an X-ray image, it is not meant to be limited to this particular type of image.

To reduce the amount of scattering of the X-rays as they pass through the object, an anti-scatter grid is sometimes placed between the object and detector (e.g. the film). This technique is well known and commonly used but can introduce some artefacts in the image. These artefacts especially appear when displaying the image on soft-copy while zooming out the image.

To perform the detection method according the described embodiment of the present invention, the digitized image data is loaded into main memory of a computer or an image processor (Step 10). Before extraction of any features, preferably first a minimum $th_{min}$ and maximum threshold value $th_{max}$ is computed to identify over-exposed and collimation areas. Techniques described in EP 610 605, EP 742 536 and EP 887 769 can be used for this purpose. Regions with pixel values outside these thresholds can then be discarded as valid input. This is shown in step 110 and step 210.

After this first rough segmentation we try to locate at various positions in both directions the positions at which the grid is most likely visible. We compute a measure for an image region. This measure is computed at different image locations. In a later stage of the algorithm we discard the image locations for which this measure is not better than an estimate of the measure if no grid was present (Step 320).

In the described implementation, this estimate is the average of the measures computed from the perpendicular direction (Step 300-310). The decision of the direction of the grid is done in step 300. In this implementation, we choose the direction for which the measure has the best score.

In the next paragraphs we describe which measure is implemented. Remark that regions which lie in the vertical direction serve to detect grids which lie horizontally. In the current implementation an image region is defined as a rectangle with width W and length L, positioned horizontally or vertically. We do not investigate image regions which lie at the border with width w and length 1 of the image.

For detection of a horizontal grid, we extract regions for several positions x with width W and length L at different positions y which contain the pixels lying in the rectangle with corner points (x,y) (x+W,y) (x+W,y+L) (x,y+L) for a vertical region (Step 100) and corner points (x,y) (x+L,y) (x+L,y+W) (x,y+W) for a horizontal region (Step 200). If any of the pixel values lies outside the threshold interval of step 20, this region is not used for inspection and an invalid measure is stored in an array of measure for this particular x column (Step 110 and 210). For the different y positions, the best measure is appended to the measures for the horizontal grid direction and the corresponding (x,y) position is saved (Step 170 and Step 270).

The measure at position (x,y) is computed in the following manner. First a high pass filter is used to remove the low frequencies from the region R (Step 120 and 220). Application of a generic simple filter with kernel elements [0.05 0.25 -0.6 0.25 0.05] is sufficient but other filters work also. The filtering is done along the length direction of the region. After this high pass filter, the mean of the filtered region is computed along the width of the region (Step 120 and 220).

This removes the noise for a great deal and reduces the impact of the noise at higher frequencies in the Fourier spectrum. Then we compute the autocorrelation of this mean profile (Step 130 and 230). The autocorrelation enhances the periodic variation in the signal.

The result of the autocorrelation is divided by its maximum value (Step 130 and 230) and the maximum of the magnitude of the Fourier transform of this scaled auto-correlated signal is computed (Step 140 and 240) and stored as measure for position (x,y) for the used direction (Step 150 and 250).

If the measures for both directions are computed, we check if the maximum measure of the horizontal direction is greater than the maximum measure of the vertical direction (Step 300). If this condition is true, we assume that the grid lies horizontally and store the positions and measure for the horizontal direction. We also compute a threshold *thresh* which represents the mean maximum power spectrum in a direction of the image without grid. This is obtained by averaging the obtained measure for the vertical direction.

If the equation of step 300 does not hold, we assume that the grid lies vertically and store positions and measure for this direction and compute the threshold from the horizontal measures.

The first feature for our classification algorithm now becomes the ratio of the measures in the assumed direction which are larger than the computed threshold over the total number of valid measures in the assumed direction (Step 310). This gives us a number between 0 and 1, which is a measure of confidence if the grid is present in the image or not. However, this feature alone is generally not sufficient enough to determine the presence of the grid.

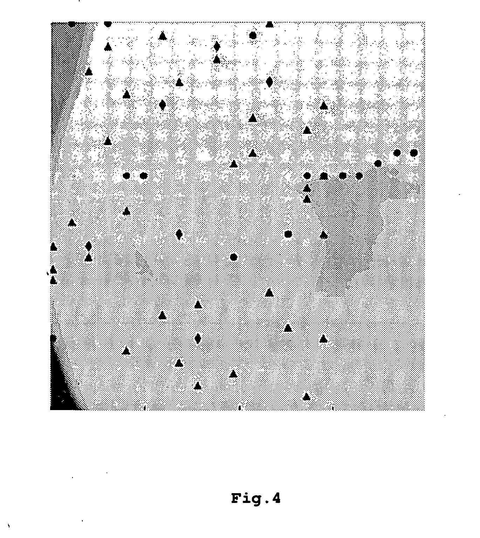

Figure 4 shows some results on an image with a horizontal grid. The circles represent positions for which the measures are higher than the mean of the measures for the vertical grid. The diamonds are positions for which this comparison does not hold. The black dots are the positions from which the threshold is computed. In this example, the feature nelem of step 310 becomes 0.823. This high value indicates that we are relatively sure there is a horizontal grid present in the image.

After removing the positions for which the measure is lower than the threshold of step 300-310, we continue to step 400 for the extraction of extra features to feed into our classification algorithm.

In step 510 or 520, again we extract regions from the image but now we only do this for the positions of step 320, for which we think the grid is most visible. This region is averaged along the direction of the grid (Step 510 or 520).

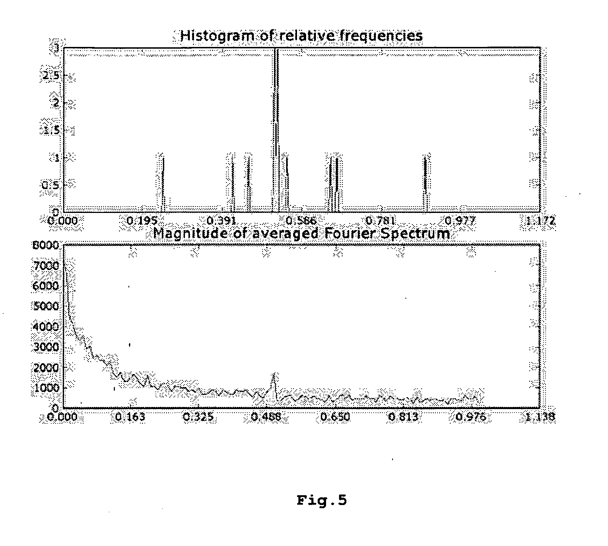

For each input position, we accumulate the Fourier spectrum F (step 600 and 610) and increase an entry of a histogram at the relative frequency f for which the amplitude of the Fourier spectrum of the auto-correlated high pass filtered input profile is maximal (steps 700 to 720). The result of both steps for the image of figure 4 is displayed in figure 5. In step 900, we search the entry in the histogram with the maximum value. This is the dominant relative frequency $\varphi$ of the grid which is present in the image. The period defined as

$$p = \frac{2}{\varphi}$$

is stored as a classification feature.

In step 910 we accumulate all neighbouring values $\lambda$ of the frequency $\varphi$ of step 900 until we reach an entry which is zero and compute the ratio to the total entries in the histogram.

In the example of figure 5, we obtain a value of 7 out of 14 entries. The count feature for this example is 0.5. In step 920, we also perform a median filter on the averaged Fourier spectrum and compute the ratio to the original spectrum (Step 930). In Step 940, we search the maximum ratio at the relative frequencies $\lambda$ which contributed to the count feature in step 910.

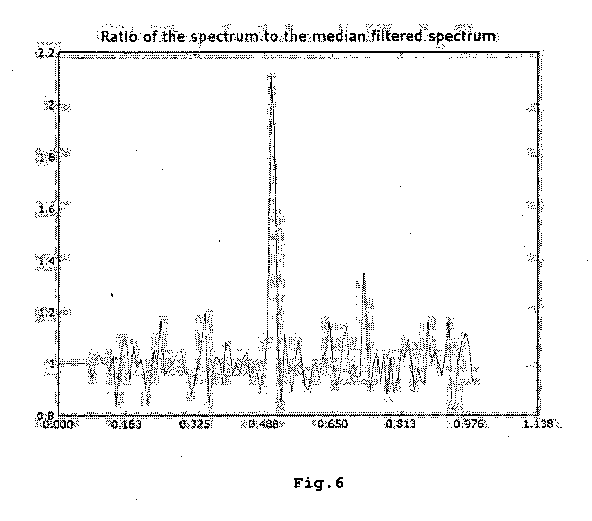

Figure 6 shows the ratio of the spectrum and the median filtered spectrum. In our example, the last feature r becomes 2.10.

Thus, one of the features is the ratio of the magnitude of the average Fourier spectrum at different image positions at the grid frequency to the magnitude of a low pass filtered averaged Fourier spectrum.

Another feature is the ratio of the number of image positions where the maximum magnitude of the Fourier spectrum is located at the grid frequency and the number of image regions where the maximum frequency of the Fourier spectrum is not located at the grid frequency.

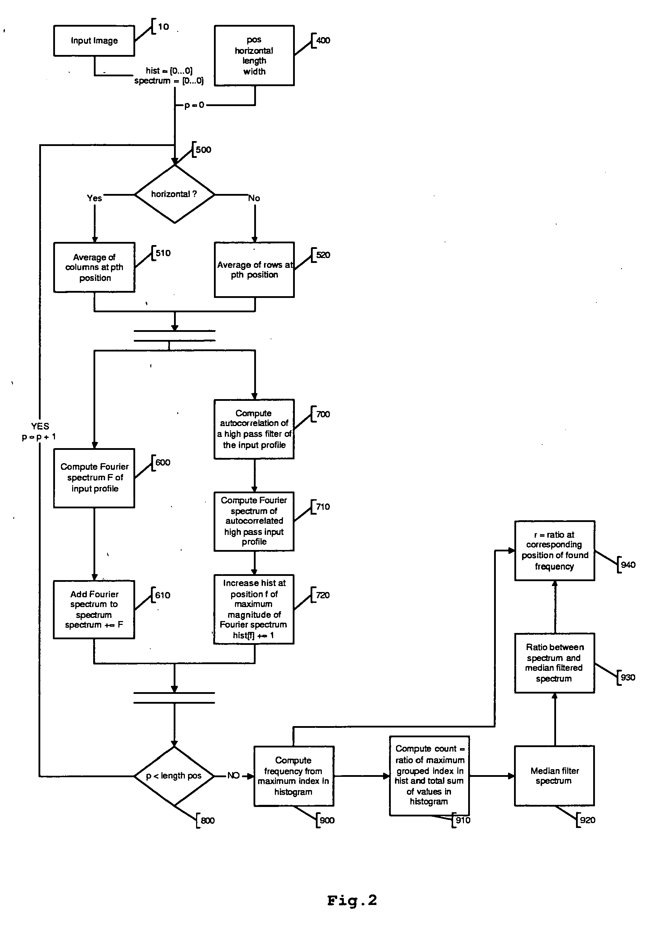

As an additional step, we can also compute the harmonics or corresponding low frequencies at which the grid manifests itself in the image. First of all, we have the ground frequency $\varphi$ obtained in the algorithm depicted in figure 2 and a corresponding ratio entry. From this value, we compute a new threshold for the ratio.

$$th = \max\left(th_{min}, \min\left(th_{max}, (r-1)*perc+1\right)\right)$$

$th_{min}$ is the predefined minimum value for the ratio, $th_{max}$ is the predefined maximum value for the ratio, *perc* is a predefined value between 0 and 1.

We define new scales and intervals on which we find new relative frequencies for which the maximum value of the ratio in the defined intervals are greater than *th*. If for a given scale and range, the following equation holds, the new relative frequency scale*f is added to frequencies we want to suppress the frequency $\gamma$ satisfying the following conditions:

$$ratio[\gamma] = \max(ratio[scale*\varphi*L-range, scale*\varphi*L+range])$$
$$ratio[\gamma] > th$$

In some conditions, the grid manifests itself in a lower frequency around the relative frequency$(1-\varphi)$. We also want to suppress the following low frequencies h

$$ratio[\eta] = \max(ratio[scale*(1-\varphi)*L-range, scale*(1-\varphi)*L+range])$$
$$ratio[\eta] > th$$

with scale equal to 1 or 2.

Having defined all these features, we are now able to train our classification algorithm.

From a number of images (also referred to as reference images) stored in advance in a database, we extract the above mentioned features and supply them as input together with the information which of the images contain grids. The choice of the classification algorithm is free.

We use cross validation against reference images from our database to choose the best classification parameters for a support vector machine implementation preferably with linear basis functions because of the simplicity. However, other basis functions such as radial basis functions are also applicable.■

**Claims**

1.  Method of detecting the presence of an image of a linear grid parallel to one of the edges of an image represented by a digital image representation comprising the steps of

    - extracting the values of a number of classification features from said digital image;
    - classifying the extracted values of the features in a classification algorithm to detect the presence of a grid
    **characterized in that**
    - said classification algorithm is trained with said classification features extracted from a number of reference images, and
    - one of said features is a confidence feature obtained by the steps of
    - computing values of a measure of likeliness of the presence of a grid for different locations in said image,
    - determining an assumed direction of said grid by comparing the values of said measure for a first and a second direction,
    - deducing a reference value from the values of said measure in the direction perpendicular to said assumed grid direction,

- calculating said confidence feature as the ratio of the number of values of said measure in the assumed grid direction which are larger than said reference value to the total number of values of said measure in the assumed grid direction.

2. A method according to claim 1 wherein said assumed direction of the grid is determined by

- computing the maximum of the measure values for said first and said second direction, and
- if the maximum of the measure values for said first direction is greater than the maximum of the measure values for said second direction, assume that said first direction is the grid direction, else assume that said first direction is the grid direction.

3. Method according to claim 1 wherein said measure is the maximum of the magnitude of the Fourier spectrum of a high passed image region.

4. Method according to claim 1 wherein said measure is the maximum of an averaged high pass filtered image region.

5. Method according to claim 1 wherein said measure is the maximum of an averaged high passed scaled auto-correlated pixel values in an image region.

6. A method according to claim 1 wherein regions in said digital image for which said measure performs worse than an estimate of said measure are discarded.

7. Method according to claim 1 wherein parts of said digital image representing collimated areas are discarded,

8. Method according to claim 1 wherein parts of said digital image representing overexposed areas are discarded.

9. Method according to claim 1 wherein said classification algorithm is implemented with a support vector machine.

10. Method according to claim 9 wherein the kernel function of said support vector machine is a linear basis function.

11. Method according to claim 1 wherein optimal values of classification parameters of said classification algorithm are obtained by optimizing said classification parameters by cross-validation against said classification parameters extracted from said reference images.

12. Method according to claim 1 wherein one of said features is the period of said grid.

13. Method according to claim 12 wherein said period of the grid is determined as the period where the corresponding entry in a histogram of frequencies at the maximum magnitudes of the Fourier spectra at different image regions where said measure is better than said estimate, is maximal.

14. Method according to claim 12 wherein one of said features is the ratio of the magnitude of the average Fourier spectrum at different image positions at the grid frequency to the magnitude of the low pass filtered averaged Fourier spectrum.

15. Method according to claim 12 wherein one of said features is the ratio of the number of image positions where the maximum magnitude of the Fourier spectrum is located at the grid frequency and the number of image positions where the maximum frequency of the Fourier spectrum is not located at the grid frequency.

16. Method according to claim 15 wherein the Fourier spectrum of an image region is computed after averaging the image region along the grid direction.

17. Method according to claim 15 wherein the Fourier spectrum of an image region is computed after high-pass filtering the averaged image region.

18. Method according to claim 17 wherein the Fourier spectrum of an image region is computed after auto correlating the high-pass filtered averaged image region.

**19.** Method according to any of the preceding claims wherein said digital image represents a medical image and said grid is an anti-scatter grid.

**Patentansprüche**

**1.** Ein Verfahren zum Prüfen des Vorhandenseins eines Bildes eines linearen Gitters, das parallel zu einer der Kanten eines durch eine digitale Bilddarstellung dargestellten Bildes verläuft, wobei das Verfahren folgende Schritte umfasst :

- Extraktion der Werte einer Anzahl von Klassifizierungskennzeichen aus dem digitalen Bild,
- Klassifizierung der extrahierten Werte der Kennzeichen in einen Klassifizierungsalgorithmus, um das Vorhandensein eines Gitters zu prüfen,

**dadurch gekennzeichnet, dass :**

- der Klassifizierungsalgorithmus mit den aus einer Anzahl von Referenzbildern extrahierten Klassifizierungskennzeichen trainiert wird, und
- eines der Kennzeichen ein durch folgende Schritte erhaltenes Sicherheitswahrscheinlichkeitskennzeichen ist :

- Berechnung von Werten eines Maßes der Wahrscheinlichkeit des Vorhandenseins eines Gitters für verschiedene Stellen im Bild,
- Ermittlung einer angenommenen Richtung des Gitters durch Vergleichen der Werte des Maßes für eine erste und eine zweite Richtung,
- Ableitung eines Referenzwertes aus den Werten des Maßes in die senkrecht zur angenommenen Gitterrichtung verlaufende Richtung, und
- Berechnung des Sicherheitswahrscheinlichkeitskennzeichens als das Verhältnis der Anzahl von Werten des Maßes in der angenommenen Gitterrichtung, die größer sind als der Referenzwert, zur Gesamtanzahl von Werten des Maßes in der angenommenen Gitterrichtung.

**2.** Verfahren nach Anspruch 1, **dadurch gekennzeichnet, dass** die angenommene Gitterrichtung des Gitters durch folgende Schritte ermittelt wird :

- Berechnung des Maximums der Maßwerte für die erste und die zweite Richtung, und
- falls das Maximum der Maßwerte für die erste Richtung größer ist als das Maximum der Maßwerte für die zweite Richtung, annehmen, dass die erste Richtung die Gitterrichtung ist, sonst annehmen, dass die zweite Richtung die Gitterrichtung ist.

**3.** Verfahren nach Anspruch 1, **dadurch gekennzeichnet, dass** das Maß die maximale Größe des Fourier-Spektrums eines hochpassgefilterten Bildbereichs ist.

**4.** Verfahren nach Anspruch 1, **dadurch gekennzeichnet, dass** das Maß das Maximum eines gemittelten hochpassgefilterten Bildbereichs ist.

**5.** Verfahren nach Anspruch 1, **dadurch gekennzeichnet, dass** das Maß das Maximum gemittelter hochpassgefilterter skalierter autokorrelierter Pixelwerte in einem Bildbereich ist.

**6.** Verfahren nach Anspruch 1, **dadurch gekennzeichnet, dass** Bereiche im digitalen Bild, für die das Maß schlechtere Ergebnisse liefert als eine Schätzung des Maßes, entfernt werden.

**7.** Verfahren nach Anspruch 1, **dadurch gekennzeichnet, dass** Teile des digitalen Bildes, die kollimierte Bereiche darstellen, entfernt werden.

**8.** Verfahren nach Anspruch 1, **dadurch gekennzeichnet, dass** Teile des digitalen Bildes, die überbelichtete Bereiche darstellen, entfernt werden.

**9.** Verfahren nach Anspruch 1, **dadurch gekennzeichnet, dass** der Klassifizierungsalgorithmus mit einer "Support Vector Machine" (SVM) implementiert wird.

**10.** Verfahren nach Anspruch 9, **dadurch gekennzeichnet, dass** die Kernelfunktion des "Support Vector Machine" eine lineare Basisfunktion ist.

**11.** Verfahren nach Anspruch 1, **dadurch gekennzeichnet, dass** optimale Werte der Klassifizierungsparameter des Klassifizierungsalgorithmus durch Optimierung der Klassifizierungsparameter durch Kreuzvalidierung gegen die aus den Referenzbildern extrahierten Klassifizierungsparameter erhalten werden.

**12.** Verfahren nach Anspruch 1, **dadurch gekennzeichnet, dass** eines der Kennzeichen die Periode des Gitters ist.

**13.** Verfahren nach Anspruch 12, **dadurch gekennzeichnet, dass** die Periode des Gitters als die Periode ermittelt wird, in der die entsprechende Eingabe in ein Histogramm von Frequenzen bei den maximalen Größen der Fourier-Spektren in verschiedenen Bildbereichen, in denen das Maß besser ist als der Schätzwert, maximal ist.

**14.** Verfahren nach Anspruch 12, **dadurch gekennzeichnet, dass** eines der Kennzeichen das Verhältnis der Größe des mittleren Fourier-Spektrums in verschiedenen Bildpositionen bei der Gitterfrequenz zur Größe des tiefpassgefilterten gemittelten Fourier-Spektrums ist.

**15.** Verfahren nach Anspruch 12, **dadurch gekennzeichnet, dass** eines der Kennzeichen das Verhältnis ist der Anzahl von Bildpositionen, an denen die maximale Größe des Fourier-Spektrums bei der Gitterfrequenz liegt, zur Anzahl von Bildpositionen, an denen die maximale Frequenz des Fourier-Spektrums nicht bei der Gitterfrequenz liegt.

**16.** Verfahren nach Anspruch 15, **dadurch gekennzeichnet, dass** das Fourier-Spektrum eines Bildbereichs nach Mittelung des Bildbereichs in die Gitterrichtung berechnet wird.

**17.** Verfahren nach Anspruch 15, **dadurch gekennzeichnet, dass** das Fourier-Spektrum eines Bildbereichs nach Hochpassfilterung des gemittelten Bildbereichs berechnet wird.

**18.** Verfahren nach Anspruch 17, **dadurch gekennzeichnet, dass** das Fourier-Spektrum eines Bildbereichs nach Autokorrelierung des hochpassgefilterten gemittelten Bildbereichs berechnet wird.

**19.** Verfahren nach einem der vorstehenden Ansprüche, **dadurch gekennzeichnet, dass** das digitale Bild ein medizinisches Bild darstellt und das Gitter ein Streustrahlenraster ist.

**Revendications**

**1.** Un procédé pour la vérification de la présence d'une image d'une grille linéaire parallèle à l'un des bords d'une image représentée par une représentation par signaux numériques, ledit procédé comprenant les étapes ci-après :

    - l'extraction des valeurs d'un nombre de caractéristiques de classification à partir de l'image numérique,
    - la classification des valeurs extraites des caractéristiques dans un algorithme de classification afin de vérifier la présence d'une grille,

    **caractérisé en ce que :**

    - l'algorithme de classification est entrainé avec les caractéristiques de classification extraites d'un nombre d'images de référence, et
    - l'une des caractéristiques est une caractéristique de confiance de probabilité, obtenue par les étapes ci-après :

        - le calcul de valeurs d'une mesure de probabilité de la présence d'une grille pour différents endroits dans l'image,
        - la détermination d'un sens supposé de la grille en comparant les valeurs de la mesure pour un premier sens et un deuxième sens,
        - la déduction d'une valeur de référence à partir des valeurs de la mesure dans le sens perpendiculaire au sens de la grille supposé, et
        - le calcul de la caractéristique de confiance de probabilité comme le rapport du nombre de valeurs de la mesure dans le sens de grille supposé qui sont supérieures à la valeur de référence, au nombre total de valeurs de la mesure dans le sens de grille supposé.

**2.** Procédé selon la revendication 1, **caractérisé en ce que** le sens supposé de la grille est déterminé par les étapes ci-après :

- le calcul du maximum des valeurs de la mesure pour le premier sens et le deuxième sens, et
- si le maximum des valeurs de la mesure pour le premier sens est supérieur au maximum des valeurs de la mesure pour le deuxième sens, supposer que le premier sens est le sens de la grille, sinon supposer que le deuxième sens est le sens de la grille.

**3.** Procédé selon la revendication 1, **caractérisé en ce que** la mesure est l'ampleur maximale du spectre Fourier d'une zone d'image filtrée passe-haut.

**4.** Procédé selon la revendication 1, **caractérisé en ce que** la mesure est le maximum d'une zone d'image filtrée passe-haut moyenne.

**5.** Procédé selon la revendication 1, **caractérisé en ce que** la mesure est le maximum de valeurs de pixel autocorrélées mises à l'échelle filtrées passe-haut moyennes dans une zone d'image.

**6.** Procédé selon la revendication 1, **caractérisé en ce que** l'on élimine des zones dans l'image numérique pour lesquelles la performance de la mesure est inférieure à une estimation de la mesure.

**7.** Procédé selon la revendication 1, **caractérisé en ce que** l'on élimine des parties de l'image numérique comprenant des zones collimatées.

**8.** Procédé selon la revendication 1, **caractérisé en ce que** l'on élimine des parties de l'image numérique comprenant des zones surexposées.

**9.** Procédé selon la revendication 1, **caractérisé en ce que** l'algorithme de classification est implémenté à l'aide d'une machine à vecteurs de support.

**10.** Procédé selon la revendication 9, **caractérisé en ce que** la fonction noyau de la machine à vecteurs de support est une fonction de base linéaire.

**11.** Procédé selon la revendication 1, **caractérisé en ce que** des valeurs optimales des paramètres de classification de l'algorithme de classification sont obtenues par optimisation des paramètres de classification en effectuant une validation croisée par rapport aux paramètres de classification extraits des images de référence.

**12.** Procédé selon la revendication 1, **caractérisé en ce que** l'une des caractéristiques est la période de la grille.

**13.** Procédé selon la revendication 12, **caractérisé en ce que** la période de la grille est déterminée comme la période dans laquelle l'entrée correspondante dans un histogramme de fréquences aux ampleurs maximales des spectres Fourier dans différentes zones de l'image dans laquelle la mesure est supérieure à l'estimation, est maximale.

**14.** Procédé selon la revendication 12, **caractérisé en ce que** l'une des caractéristiques est le rapport de l'ampleur du spectre Fourier moyen à différentes positions d'image à la fréquence de la grille, à l'ampleur du spectre Fourier filtré passe-bas moyen.

**15.** Procédé selon la revendication 12, **caractérisé en ce que** l'une des caractéristiques est le rapport du nombre de positions d'image où l'ampleur maximale du spectre Fourier se situe à la fréquence de la grille, au nombre de positions d'image où la fréquence maximale du spectre Fourier ne se situe pas à la fréquence de la grille.

**16.** Procédé selon la revendication 15, **caractérisé en ce que** le spectre Fourier d'une zone d'image est calculé après la détermination de la moyenne de la zone d'image dans le sens de la grille.

**17.** Procédé selon la revendication 15, **caractérisé en ce que** le spectre Fourier d'une zone d'image est calculé après le filtrage passe-haut de la zone d'image moyenne.

**18.** Procédé selon la revendication 17, **caractérisé en ce que** le spectre Fourier d'une zone d'image est calculé après l'autocorrélation de la zone d'image moyenne filtrée passe-haut.

**19.** Procédé selon l'une quelconque des revendications précédentes, **caractérisé en ce que** l'image numérique représente une image médicale et que la grille est une grille antidiffusante.

Fig.1

Fig.2

Extract
nelem,r,count and
frequency for a
database of known
images

Extract
nelem,r,count and
frequency of an
unknown image

Load classification
rules

Train classifier

Classify

Store classification
rules

**Fig.3**

Fig.4

Fig.5

Ratio of the spectrum to the median filtered spectrum

Fig.6

**EP 1 696 366 B1**

**Patent documents cited in the description**

- US 6269176 B1
- EP 0723762 A
- EP 610605 A
- EP 742536 A
- EP 887769 A